# EUROPEAN PATENT APPLICATION

(11) **EP 3 537 453 A1**
(43) Date of publication of application: **11.09.2019**
(21) Application number: 18159926.7
(22) Date of filing: 05.03.2018
(51) Int. Cl.: G16H 40/67, G16H 20/10, G16H 20/30

(54) **HAND HELD COMMUNICATION DEVICE**

(71) Applicant: Bayer AG, 51373 Leverkusen (DE)
(72) Inventor: Kilic, Levent, 12101 Berlin (DE); Rakutt, Wolf-Dieter, 13467 Berlin (DE)
(74) Representative: BIP Patents

(57) **Abstract**

The present invention relates to a hand held communication device. It is described to provide (310) information relating to a patient to a hand held communication device. A communication link is established (320) between the hand held communication device and an apparatus associated with the patient comprising utilization of the information relating to the patient. The hand held communication device receives (330) operational data associated with operation of a medical device by the patient. Receiving the operational data involves receiving (332) compliance data over the communication link from the apparatus relating to utilization of the medical device for a medical condition of the patient.

## Description

### FIELD OF THE INVENTION

The present invention relates to a hand held communication device, an apparatus associated with a patient, a system for capturing operational data associated with operation of a medical device, and a method for receiving operational data associated with operation of a medical device, as well as to a computer program element and a computer readable medium.

### BACKGROUND OF THE INVENTION

The general background of this invention is the collection of adherence data relating to the degree to which a patient correctly follows medical advice for the utilization of a medical device for a medical condition. World-wide, non-compliance is a major obstacle to the effective delivery of health care. Only about 50% of patients with chronic diseases living in developed countries follow treatment recommendations, with particularly low rates for asthma, diabetes, and hypertension. Frequently, medical devices need to be used by patients as part of the treatment of the medical condition. However, determining and obtaining adherence data for a patient's use of a medical device is problematic.

### SUMMARY OF THE INVENTION

It would be advantageous to provide means for determining and acquiring adherence data for a patient's use of a medical device for a medical condition.

The object of the present invention is solved with the subject matter of the independent claims, wherein further embodiments are incorporated in the dependent claims. It should be noted that the following described aspects and examples of the invention apply also for the hand held communication device, the apparatus associated with a patient, the system for capturing operational data associated with operation of a medical device, and the method for receiving operational data associated with operation of a medical device, and for the computer program element and the computer readable medium.

According to a first aspect, there is provided a hand held communication device, comprising:
- an input unit;
- a processing unit; and
- a transceiver.

The input unit is configured to enable a user of the device to provide information relating to a patient. The processing unit is configured to establish a communication link to an apparatus associated with the patient comprising utilization of the information relating to the patient and the transceiver. The processing unit is configured to utilize the transceiver to receive operational data associated with operation of a medical device by the patient. The operational data comprises compliance data received over the communication link from the apparatus, wherein the compliance data relates to utilization of the medical device for a medical condition of the patient.

In this manner, compliance data relating to utilization of a medical device of a patient for a medical condition of a patient can be easily and efficiently received, for example wirelessly, simply on the basis of a user inputting information relating to a patient. Thus a patient support partner (PSP) visiting patients at their home or at hospital can easily and efficiently acquire compliance data relating to a patient's utilization of a medical device on the basis for example of selecting a patient's name from a list of patients on their hand held communication device such as a mobile telephone within an appropriate App. The patient's name is tagged with information relating to an apparatus used by the patient that stores the compliance data, enabling a link to be established between the PSP's hand held communication device and the patient's apparatus in order that the compliance data is downloaded to the PSP's hand held communication device.

Thus, compliance data relating to utilization of the medical device can be downloaded directly from the medical device to the PSP's hand held communication device, in which case the apparatus is the medical device. Or, the compliance data can be downloaded from the medical device to another device, and then subsequently downloaded from that device to the hand held communication device. Thus, a communication module associated with one or more medical devices, for example at a hospital or clinic, can automatically have downloaded to it data acquired from a medical device relating to the utilization of that device by a patient, and then a PSP can easily acquire that data from the communication module.

In an example, the apparatus associated with the patient is the medical device.

In an example, the apparatus associated with the patient is configured to receive the operational data associated with operation of the medical device by the patient from the medical device.

In other words, the apparatus acquires the operational data from the medical device that is then downloaded to the user's hand held communication device on the basis of information relating to the patient. Thus for example, a communication module associated with the medical device can acquire data from the medical device. This data is then downloaded to a user's hand held communication device on the basis of information relating to the patient.

In an example, the operational data comprises activity data of the patient relating to times of utilization of the medical device for the medical condition of the patient.

In this way medical service providers can be provided not only with compliance data associated with a patient's utilization of a medical device, but can also be provided with activity information. Thus, if a patient becomes sedentary at a time when compliance for a medical treatment drops, a physician can take this into account when talking to the patient.

In other words, data imported from an activity tracker can be analysed to determine if there is a correlation between adherence of usage of a medical device and activity levels, providing a medical practitioner with further potentially important information.

For example, this could indicate that the patient has another underlying problem or condition or issue that is leading to or influencing non-compliance and the medical practitioner or physician can take this into account.

In an example, the processing unit is configured to establish a second communication link to an activity tracking device of the patient comprising utilization of the information relating to the patient and the transceiver. The operational data can then comprise the activity data of the patient received over the second communication link from the activity tracking device relating to times of utilization of the medical device for the medical condition of the patient.

In an example, the apparatus associated with the patient is configured to receive the activity data of the patient from an activity tracking device of the patient.

In an example, the operational data comprises happiness level data of the patient relating to times of utilization of the medical device for the medical condition of the patient.

In an example, the processing unit is configured to establish a third communication link to a communication device of the patient comprising utilization of the information relating to the patient and the transceiver. The communication device is configured to receive information relating to a degree of happiness of the patient from which the happiness level data are derived. The operational data can then comprise the happiness level data of the patient received over the third communication link from the communication device of the patient relating to times of utilization of the medical device for the medical condition of the patient.

In an example, the apparatus associated with the patient is configured to receive the happiness level data of the patient from a communication device of the patient.

In an example, the processing unit is configured to establish a fourth communication link with a health care provider server comprising utilization of the transceiver. The processing unit is configured to utilize the transceiver to the send to the health care provider server the operational data associated with operation of the medical device by the patient in association with information derived from the information relating to the patient.

According to a second aspect, there is provided an apparatus associated with a patient, comprising:
- an input unit;
- a processing unit; and
- a transceiver.

The input unit is configured to enable the apparatus to acquire compliance data relating to utilization of a medical device by the patient for a medical condition of the patient. The processing unit is configured to establish a communication link to a hand held communication device of a user on the basis of information received from the hand held communication device comprising utilization of the transceiver. The processing unit is configured to utilize the transceiver to send the compliance data relating to utilization of the medical device by the patient over the communication link to the hand held communication device.

In an example, the apparatus is the medical device.

In an example, the apparatus is configured to receive activity data of the patient received from an activity tracking device relating to times of utilization of the medical device for the medical condition of the patient.

In an example, the apparatus is configured to receive happiness level data of the patient from a communication device of the patient relating to times of utilization of the medical device for the medical condition of the patient.

According to a third aspect, there is provided a system for capturing operational data associated with operation of a medical device, comprising:
- a hand held communication device according to the first aspect; and
- an apparatus associated with a patient according to the second aspect.

The hand held communication device is configured to receive operational data associated with operation of a medical device of the patient from the apparatus. The operational data comprises compliance data received over a communication link between the hand held communication device and the apparatus. The compliance data relates to utilization of the medical device for a medical condition of the patient.

According to a fourth aspect, there is provided a method for receiving operational data associated with operation of a medical device, comprising:
d) providing information relating to a patient to a hand held communication device;
e) establishing a communication link between the hand held communication device and an apparatus associated with the patient comprising utilization of the information relating to the patient;
h) receiving by the hand held communication device operational data associated with operation of a medical device by the patient, wherein receiving the operational data comprises:
   h1) receiving compliance data over the communication link from the apparatus relating to utilization of the medical device for a medical condition of the patient.

According to another aspect, there is provided a computer program element for controlling an device according to the first aspect and/or apparatus according to the second aspect and/or system according to the third aspect, which when executed by one or more processors is configured to carry out the method of the fourth aspect.

According to another aspect, there is provided a computer readable medium having stored the program element.

Advantageously, the benefits provided by any of the above aspects equally apply to all of the other aspects and vice versa.

The above aspects and examples will become apparent from and be elucidated with reference to the embodiments described hereinafter.

### BRIEF DESCRIPTION OF THE DRAWINGS

Exemplary embodiments will be described in the following with reference to the following drawings:
Fig. 1 shows a schematic set up of an example of a hand held communication device;
Fig. 2 shows a schematic set up of an example of an apparatus associated with a patient;
Fig. 3 shows a schematic set up of an example of a system for capturing operational data associated with operation of a medical device;
Fig. 4 shows a method for receiving operational data associated with operation of a medical device;
Fig. 5 shows an example of a hand held communication device;
Fig. 6 shows an example of a system for capturing operational data associated with operation of a medical device; and
Fig. 7 shows an example of operational data associated with operation of a medical device.

### DETAILED DESCRIPTION OF EMBODIMENTS

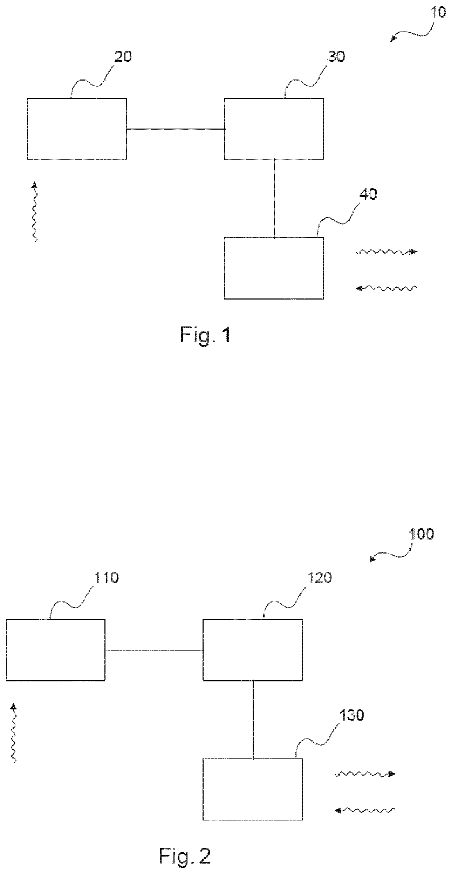

Fig. 1 shows an example of a hand held communication device 10. The hand held communication device 10 comprises an input unit 20, a processing unit 30 and a transceiver 40. The input unit 20 is configured to enable a user of the device 10 to provide information relating to a patient. The processing unit 30 is configured to establish a communication link to an apparatus associated with the patient comprising utilization of the information relating to the patient and the transceiver 40. The processing unit 30 is configured to utilize the transceiver 40 to receive operational data associated with operation of a medical device by the patient. The operational data comprises compliance data received over the communication link from the apparatus. The compliance data relates to utilization of the medical device for a medical condition of the patient.

In an example, the hand held communication device is a mobile phone.

In an example, the medical device is an inhaler.

In an example, the medical device is a nebulizer.

In an example, the medical device is an insulin providing device.

In an example, the operational data associated with operation of the medical device comprises at least one date of operation of the medical device.

In an example, the operational data associated with operation of the medical device comprises at least one duration of operation of the medical device.

In an example, the operational data associated with operation of the medical device comprises at least one indication of whether utilization of the medical device for the medical condition of the patient is complete for an associated at least one operation of the medical device.

In an example, the operational data associated with operation of the medical device comprises at least one indication of whether utilization of the medical device for the medical condition of the patient is incomplete for an associated at least one operation of the medical device.

According to an example, the apparatus associated with the patient is the medical device.

According to an example, the apparatus associated with the patient is configured to receive the operational data associated with operation of the medical device by the patient from the medical device.

According to an example, the operational data comprises activity data of the patient relating to times of utilization of the medical device for the medical condition of the patient.

According to an example, the processing unit is configured to establish a second communication link to an activity tracking device of the patient comprising utilization of the information relating to the patient and the transceiver. The operational data received by the hand held communication device can then comprise the activity data of the patient received over the second communication link from the activity tracking device relating to times of utilization of the medical device for the medical condition of the patient.

According to an example, the apparatus associated with the patient is configured to receive the activity data of the patient from an activity tracking device of the patient.

In an example, the activity tracking device is a wrist worn activity tracker.

In an example, the activity tracking device is a mobile telephone.

According to an example, the operational data comprises happiness level data of the patient relating to times of utilization of the medical device for the medical condition of the patient.

According to an example, the processing unit is configured to establish a third communication link to a communication device of the patient comprising utilization of the information relating to the patient and the transceiver. The communication device is configured to receive information relating to a degree of happiness of the patient from which the happiness level data are derived. The operational data received by the hand held communication device can the comprise the happiness level data of the patient received over the third communication link from the communication device of the patient relating to times of utilization of the medical device for the medical condition of the patient.

According to an example, the apparatus associated with the patient is configured to receive the happiness level data of the patient from the communication device of the patient.

In an example, the communication device of the patient is a mobile telephone.

According to an example, the processing unit is configured to establish a fourth communication link with a health care provider server comprising utilization of the transceiver. The processing unit is configured to utilize the transceiver to the send to the health care provider server the operational data associated with operation of the medical device by the patient in association with information derived from the information relating to the patient.

Fig. 2 shows an example of an apparatus associated with a patient 100. The apparatus 100 comprises an input unit 110, a processing unit 120 and a transceiver 130. The input unit 110 is configured to enable the apparatus 100 to acquire compliance data relating to utilization of a medical device by the patient for a medical condition of the patient. The processing unit 120 is configured to establish a communication link to a hand held communication device of a user on the basis of information received from the hand held communication device comprising utilization of the transceiver 130. The processing unit 120 is configured also to utilize the transceiver 130 to send the compliance data relating to utilization of the medical device by the patient over the communication link to the hand held communication device.

According to an example, the apparatus is the medical device. Thus, an input unit of a medical device acquires compliance data relating to utilization of a medical device by the patient for a medical condition of the patient. A processing unit 120 of the medical device then establishes a communication link to a hand held communication device of a user on the basis of information received from the hand held communication device comprising utilization of the transceiver 130 of the medical device. The processing unit 120 then utilizes the transceiver 130 to send the compliance data relating to utilization of the medical device by the patient over the communication link to the hand held communication device.

According to an example, the apparatus (medical device) is configured to receive activity data of the patient received from an activity tracking device relating to times of utilization of the medical device for the medical condition of the patient.

According to an example, the apparatus (medical device) is configured to receive happiness level data of the patient from a communication device of the patient relating to times of utilization of the medical device for the medical condition of the patient.

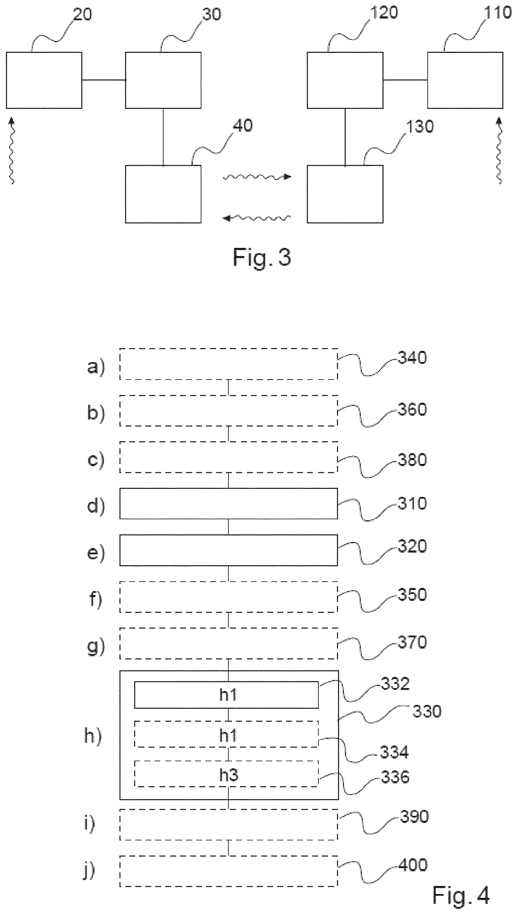

Fig. 3 shows an example of a system 200 for capturing operational data associated with operation of a medical device. The system 200 comprises a hand held communication device 10 and an apparatus 100. The hand held communication device 10 is as described with respect to any of the examples above described in association with Fig. 1. The apparatus 100 is as described with respect to any of the examples above described in association with Fig. 2. Therefore, the hand held communication device 10 is configured to receive operational data associated with operation of a medical device of the patient from the apparatus 100. As discussed above, the apparatus 100 and the medical device can be one and the same, but need not be. The operational data comprises compliance data received over a communication link between the hand held communication device 10 and the apparatus (medical device) 100. The compliance data relates to utilization of the medical device for a medical condition of the patient.

Fig. 4 shows a method 300 for receiving operational data associated with operation of a medical device in its basic steps, where dashed boxes relate to optional method steps. The method 300 comprises:
in a providing step 310, also referred to as step d), providing information relating to a patient to a hand held communication device;
in an establishing step 320, also referred to as step e), establishing a communication link between the hand held communication device and an apparatus associated with the patient comprising utilization of the information relating to the patient;
in a receiving step 330, also referred to as step h), receiving by the hand held communication device operational data associated with operation of a medical device by the patient, wherein receiving the operational data comprises:
   in a receiving step 332, also referred to as step h1), receiving compliance data over the communication link from the apparatus relating to utilization of the medical device for a medical condition of the patient.

In an example, the apparatus associated with the patient is the medical device.

In an example, the method comprises step a) receiving 340 by the apparatus the operational data associated with operation of the medical device by the patient from the medical device.

In an example, step h) comprises step h2) receiving 334 activity data of the patient relating to times of utilization of the medical device for the medical condition of the patient.

In an example, the method comprises step f) establishing 350 a second communication link to an activity tracking device of the patient comprising utilization of the information relating to the patient; and wherein step h2) comprises receiving the activity data of the patient over the second communication link from the activity tracking device.

In an example, the method comprises step b) receiving 360 by the apparatus the activity data of the patient from the activity tracking device of the patient.

In an example, step h) comprises step h3) receiving 336 happiness level data of the patient relating to times of utilization of the medical device for the medical condition of the patient.

In an example, the method comprises step g) establishing 370 a third communication link to a communication device of the patient comprising utilization of the information relating to the patient, wherein the communication device is configured to receive information relating to a degree of happiness of the patient from which the happiness level data are derived; and wherein step h3) comprises receiving the happiness level data of the patient received over the third communication link from the communication device of the patient.

In an example, the method comprises step c) receiving 380 by the apparatus the happiness level data of the patient from the communication device of the patient.

In an example, the method comprises step i) establishing 390 a fourth communication link between the hand held communication device and a health care provider server, and the method comprises step j) sending 400 from the hand held communication device to the health care provider server the operational data associated with operation of the medical device by the patient in association with information derived from the information relating to the patient.

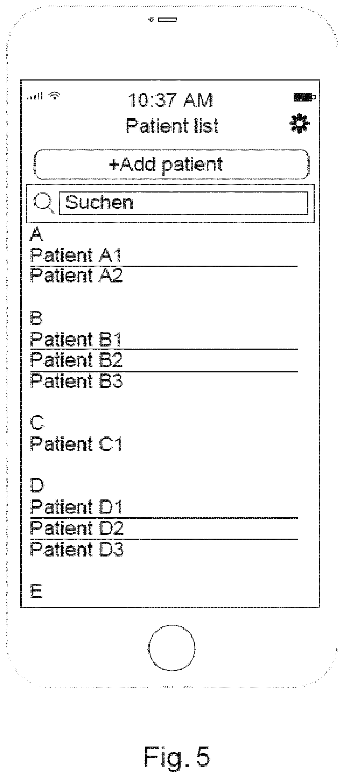

Fig. 5 shows an example of a hand held communication device as described with respect to Fig. 1. A health care service provider uses this hand held communication device when they visit a clinic or hospital, or care home or even the home of a patient. The health care serve provider wishes to acquire adherence data relating to a patient's utilization of a medical device for a medical condition. At the same time, the health care provider wishes to acquire other information that can show a correlation with compliance or non-compliance of a treatment regime requiring utilization of the medical device, such as how active the patient has been and information provider by the patient regarding how well they feel. On the hand held communication device there is a menu of patient's names, and upon selection of the relevant patient, i.e., the one being visited and for whom the above described information is wished to be acquired, the associated medical device details and activity tracking device details and device the patient has used to log their well being (or mood) is obtained from a lookup table. Then the hand haled communication device automatically links to these devices and the appropriate information is downloaded from these devices to the service care provider's hand held communication device. Rather than being downloaded directly from the medical device to the hand held communication device, in some circumstances data from the medical device is downloaded to an intermediate device or apparatus, because for instance the medical device may have limited memory and/or be used by different patients. Then, this intermediate device tags that data with the patient who had used the medical device. Then, when the health care service provide enters the patient's details as discussed above, a link or connection is made to this intermediate device and the data downloaded to the hand held communication device. The data acquired by the medical device and down loaded to the hand held communication device either directly, or via the intermediate device, contains information such as the duration of utilization of the medical device, the time and date of utilization (enabling correlation with activity data and mood data), the duration or utilization of the medical device and even a yes/no indication if a particular treatment episode has been successfully completed.

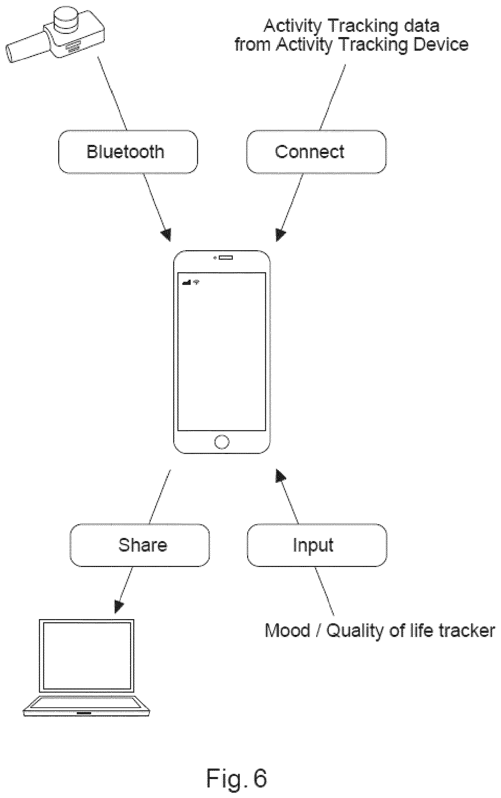

Fig. 6 shows an example of how a hand held communication device operates. The hand held communication device is shown in the centre. In the upper left is a medical device, which in this example is an inhaler, but could have been any number of different medical devices such as an insulin providing medical device. Referring to Fig. 5, the health care service provider is visiting patient B. Patient B has been using the inhaler as part of a treatment for a medical condition. They have also been wearing an activity tracking device, and agreed that the health care provider can obtain this information. Periodically, and ideally when the patient is using the medical device, the patient enters an indication of their mood on their mobile phone. They can indicate from a scale of 1-10 how they feel, with 10 meaning that they feel great, with 1 meaning that they do not feel in the best of sorts. After selecting patient B on the hand held communication device, the appropriate connections are made to these devices and the relevant data down loaded. The health care provided then selects their office details, and the downloaded information is sent to a server and tagged with the patient's details, from which it can be viewed by health care professionals.

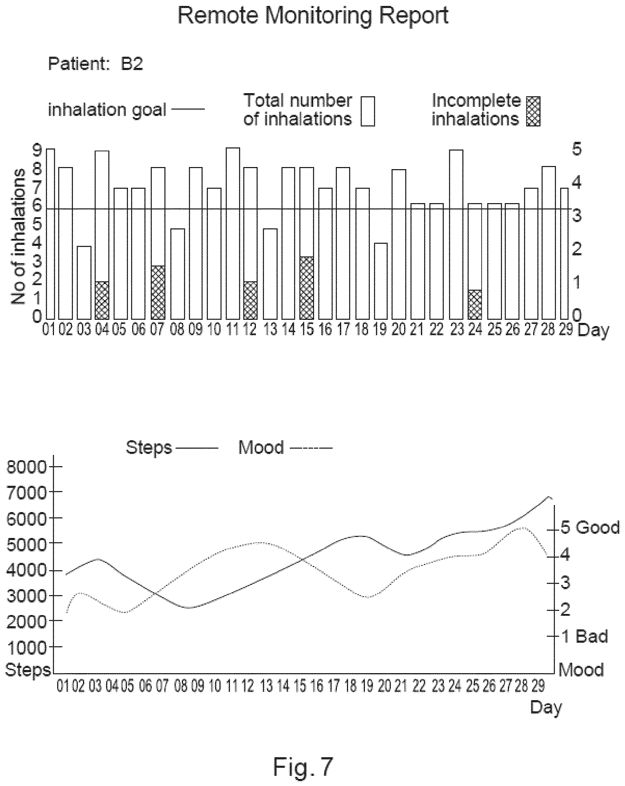

Fig. 7 shows an example of the data acquired for the specific example of patient B's use of an inhaler.

In another exemplary embodiment, a computer program or computer program element is provided that is characterized by being configured to execute the method steps of the method according to one of the preceding embodiments, on an appropriate apparatus or system.

The computer program element might therefore be stored on a computer unit, which might also be part of an embodiment. This computing unit may be configured to perform or induce performing of the steps of the method described above. Moreover, it may be configured to operate the components of the above described apparatus and/or system. The computing unit can be configured to operate automatically and/or to execute the orders of a user. A computer program may be loaded into a working memory of a data processor. The data processor may thus be equipped to carry out the method according to one of the preceding embodiments.

This exemplary embodiment of the invention covers both, a computer program that right from the beginning uses the invention and computer program that by means of an update turns an existing program into a program that uses invention.

Further on, the computer program element might be able to provide all necessary steps to fulfill the procedure of an exemplary embodiment of the method as described above.

According to a further exemplary embodiment of the present invention, a computer readable medium, such as a CD-ROM, USB stick or the like, is presented wherein the computer readable medium has a computer program element stored on it which computer program element is described by the preceding section.

A computer program may be stored and/or distributed on a suitable medium, such as an optical storage medium or a solid state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A hand held communication device (10), comprising:
- an input unit (20);
- a processing unit (30); and
- a transceiver (40);
wherein, the input unit is configured to enable a user of the device to provide information relating to a patient;
wherein, the processing unit is configured to establish a communication link to an apparatus associated with the patient comprising utilization of the information relating to the patient and the transceiver; and
wherein, the processing unit is configured to utilize the transceiver to receive operational data associated with operation of a medical device by the patient, and wherein the operational data comprises compliance data received over the communication link from the apparatus, wherein the compliance data relates to utilization of the medical device for a medical condition of the patient.

2. Device according to claim 1, wherein the apparatus associated with the patient is the medical device.

3. Device according to claim 1, wherein the apparatus associated with the patient is configured to receive the operational data associated with operation of the medical device by the patient from the medical device.

4. Device according to any of claims 1-3, wherein the operational data comprises activity data of the patient relating to times of utilization of the medical device for the medical condition of the patient.

5. Device according to claim 4, wherein, the processing unit is configured to establish a second communication link to an activity tracking device of the patient comprising utilization of the information relating to the patient and the transceiver; and wherein the operational data comprises the activity data of the patient received over the second communication link from the activity tracking device relating to times of utilization of the medical device for the medical condition of the patient.

6. Device according to claim 4, wherein the apparatus associated with the patient is configured to receive the activity data of the patient from an activity tracking device of the patient.

7. Device according to any of claims 1-6, wherein the operational data comprises happiness level data of the patient relating to times of utilization of the medical device for the medical condition of the patient.

8. Device according to any of claims 1-7, wherein, the processing unit is configured to establish a third communication link to a communication device of the patient comprising utilization of the information relating to the patient and the transceiver, wherein the communication device is configured to receive information relating to a degree of happiness of the patient from which the happiness level data are derived; and wherein the operational data comprises the happiness level data of the patient received over the third communication link from the communication device of the patient relating to times of utilization of the medical device for the medical condition of the patient.

9. Device according to claim 7, wherein the apparatus associated with the patient is configured to receive the happiness level data of the patient from a communication device of the patient.

10. Device according to any of claims 1-9, wherein the processing unit is configured to establish a fourth communication link with a health care provider server comprising utilization of the transceiver, and wherein the processing unit is configured to utilize the transceiver to the send to the health care provider server the operational data associated with operation of the medical device by the patient in association with information derived from the information relating to the patient.

11. An apparatus associated with a patient (100), comprising:
- an input unit (110);
- a processing unit (120); and
- a transceiver (130);
wherein, the input unit is configured to enable the apparatus to acquire compliance data relating to utilization of a medical device by the patient for a medical condition of the patient;
wherein, the processing unit is configured to establish a communication link to a hand held communication device of a user on the basis of information received from the hand held communication device comprising utilization of the transceiver; and
wherein, the processing unit is configured to utilize the transceiver to send the compliance data relating to utilization of the medical device by the patient over the communication link to the hand held communication device.

12. Apparatus associated with a patient according to claim 11, wherein the apparatus is the medical device.

13. Apparatus associated with a patient according to any of claims 11-12, wherein the apparatus is configured to receive activity data of the patient received from an activity tracking device relating to times of utilization of the medical device for the medical condition of the patient.

14. Apparatus associated with a patient according to any of claims 11-13, wherein the apparatus is configured to receive happiness level data of the patient from a communication device of the patient relating to times of utilization of the medical device for the medical condition of the patient.

15. A system (200) for capturing operational data associated with operation of a medical device, comprising:
- a hand held communication device (10) according to any of claims 1-10; and
- an apparatus (100) associated with a patient according to any of claims 11-14;
wherein, the hand held communication device is configured to receive operational data associated with operation of a medical device of the patient from the apparatus, and wherein the operational data comprises compliance data received over a communication link between the hand held communication device and the apparatus, wherein the compliance data relates to utilization of the medical device for a medical condition of the patient.

16. A method (300) for receiving operational data associated with operation of a medical device, comprising:
d) providing (310) information relating to a patient to a hand held communication device;
e) establishing (320) a communication link between the hand held communication device and an apparatus associated with the patient comprising utilization of the information relating to the patient; and
h) receiving (330) by the hand held communication device operational data associated with operation of a medical device by the patient, wherein receiving the operational data comprises:
h1) receiving (332) compliance data over the communication link from the apparatus relating to utilization of the medical device for a medical condition of the patient.
